# FASCICULE DE BREVET EUROPEEN

(11) **EP 0 741 793 B1**
(45) Date de publication et mention de la délivrance du brevet: **16.11.2005**
(21) Numéro de dépôt: 95907045.9
(22) Date de dépôt: 18.01.1995
(51) Int. Cl.: C12N 15/86, C12N 15/35, C12N 15/27, C12N 5/10, C12N 7/01

(54) **PROCEDE DE PREPARATION DE VIRUS ADENO-ASSOCIES (AAV) RECOMBINANTS ET UTILISATIONS**
VERFAHREN ZUR HERSTELLUNG REKOMBINANTER ADENO-ASSOCIATED VIREN (AAV) UND DEREN VERWENDUNG
METHOD FOR PREPARING RECOMBINANT ADENO-ASSOCIATED VIRUSES (AAV), AND USES THEREOF

(30) Priorité: 28.01.1994 FR 9400934
(43) Date de publication de la demande: 13.11.1996
(73) Titulaire: Aventis Pharma S.A., 92160 Antony (FR)
(72) Inventeur: DESCAMPS, Vincent, F-78160 Marly-le-Roy (FR); PERRICAUDET, Michel, F-28320 Ecrosnes (FR)
(74) Mandataire: Lecca, Patricia S.
(86) Numéro de dépôt international: PCT/FR1995/000054
(87) Numéro de publication internationale: WO 1995/020671

(56) Documents cités:
- EP-A- 0 488 528
- WO-A-91/18088
- US-A- 5 139 941
- VIROLOGY, vol.152, no.1, 15 Juillet 1986 pages 110 - 117 SCHLEHOFER, J.R. ET AL. 'Vaccinia Virus, Herpes Simplex Virus and carcinogens induce DNA amplification in a human cell line and support replication of a helper dependent parvovirus'
- VIROLOGY, vol.134, no.1, 15 Avril 1984 pages 64 - 71 GEORG-FRIES, B. ET AL. 'Analysis of proteins, helper dependence and seroepidemiology of a new human parvovirus'
- JOURNAL OF GENERAL VIROLOGY, vol.67, no.1, Janvier 1986 pages 181 - 185 BAUER, H.J. & MONREAL, G 'Herpesviruses provide helper functions for avian adeno-associated parvovirus'
- JOURNAL OF VIROLOGICAL METHODS, vol.29, no.3, Septembre 1990 pages 335 - 340 BAUER, A. ET AL. 'Growth of avian adeno-associated virus in chicken cells transfected with fowl adenovirus serotype 1 DNA'
- JOURNAL OF CELLULAR BIOCHEMISTRY, vol.SUP, no.18A, 4 Janvier 1994 page 214 CARTER, T. ET AL. 'Adeno-associated virus vectors for in vivo gene transfer' & Keystone symposium on gene therapy Copper mountain, USA 15-22 Janvier 1994
- PROCEEDINGS OF THE NATIONAL ACADEMY OF SCIENCES OF USA., vol.81, no.20, Octobre 1984, WASHINGTON US pages 6466 - 6470 HERMONAT, P. L. & MUZYCZKA, N. 'Use of adeno-associated virus as a mammalian DNA cloning vector: transduction of neomycin resistance into mammlian tissue culture cells'

## Description

La présente invention concerne une nouvelle méthode de préparation de vecteurs dérivés des virus adéno-associés (AAV) et les cellules utilisées à cet effet. Plus particulièrement, la présente invention concerne un procédé de préparation d'AAV recombinants au moyen d'un virus auxiliaire animal. L'invention concerne également l'utilisation des AAV recombinants obtenus, notamment en thérapie génique et/ou cellulaire.

La thérapie génique consiste à corriger une déficience ou une anomalie (mutation, expression aberrante, etc) ou à assurer l'expression d'une protéine d'intérêt thérapeutique par introduction d'une information génétique dans la cellule ou l'organe affecté. Cette information génétique peut être introduite soit in vitro dans une cellule extraite de l'organe, la cellule modifiée étant alors réintroduite dans l'organisme, soit directement in vivo dans le tissu approprié. Différentes techniques ont été décrites pour le transfert de cette information génétique, parmi lesquelles des techniques diverses de transfection impliquant des complexes d'ADN et de DEAE-dextran (Pagano et al., J. Virol. 1 (1967) 891), d'ADN et de protéines nucléaires (Kaneda et al., Science 243 (1989) 375), d'ADN et de lipides (Felgner et al., PNAS 84 (1987) 7413), d'ADN et de polylysine, l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physico-chimiques de transfection. A cet égard, différents virus ont été testés pour leur capacité à infecter certaines populations cellulaires. En particulier, les rétrovirus (RSV, HMS, MMS, etc), le virus HSV, les adénovirus et les virus adéno-associés.

Parmi ces virus, les virus adéno-associés (AAV, pour "adeno-associated virus"), présentent certaines propriétés attrayantes en vue d'une utilisation pour le transfert de gènes. Les AAV sont des virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme.
Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus. L'utilisation de vecteurs dérivés des AAV pour le transfert de gènes in vitro et in vivo a été décrite dans la littérature (voir notamment WO 91/18088; WO 93/09239; US 4,797,368, US5,139,941, EP 488 528). Ces demandes décrivent différentes constructions dérivées des AAV, dans lesquelles les gènes rep et/ou cap sont délétés et remplacés par un gène d'intérêt, et leur utilisation pour transférer in vitro (sur cellules en culture) ou in vivo (directement dans un organisme) ledit gène d'intérêt.
Toutefois, l'utilisation thérapeutique des AAV comme vecteurs pour le transfert de gènes est actuellement limitée, en raison notamment des risques de contamination des AAV recombinants par un virus auxiliaire sauvage, et de l'utilisation, pour la production des virus recombinants, de lignées cellulaires humaines potentiellement tumorigènes. Les AAV ont besoin, pour se répliquer, de la présence d'un virus auxiliaire ("helper"). Le terme virus auxiliaire désigne tout virus capable d'apporter en trans les fonctions nécessaires à la réplication des AAV. Il peut s'agir en particulier d'un adénovirus, d'un virus de l'herpes ou d'un virus de la vaccine. En l'absence d'un tel virus auxiliaire, les AAV restent sous forme latente dans le génome des cellules infectées, mais ne peuvent se répliquer et ainsi produire des particules virales. Généralement, les AAV recombinants sont donc produits par co-transfection, dans un lignée cellulaire infectée par un virus auxiliaire humain (par exemple un adénovirus), d'un plasmide contenant le gène d'intérêt bordé de deux régions répétées inversées (ITR) d'AAV et d'un plasmide portant les gènes d'encapsidation (gènes rep et cap) d'AAV. Les AAV recombinants produits sont ensuite purifiés par des techniques classiques. Cependant, les AAV ainsi obtenus sont généralement contaminés par du virus auxiliaire sauvage, également produit par la cellule productrice. La présence de ce virus auxiliaire peut avoir in vivo des conséquences très néfastes telles que par exemple un effet pathogène ou immunogène. Ce virus auxiliaire peut également être responsable d'événements de recombinaisons, ou induire la réplication de l'AAV recombinant et ainsi sa transmission et sa dissémination.

La présente invention apporte une solution avantageuse à ce problème. La demanderesse a en effet mis au point un procédé de production d'AAV recombinants qui permet de produire des stocks d'AAV recombinants dépourvus de virus auxiliaire humain sauvage. Le procédé de l'invention permet également d'éviter l'utilisation de cellules productrices potentiellement tumorigènes. Le procédé de l'invention repose en partie sur la mise en évidence que les AAV recombinants peuvent être produits en utilisant un virus auxiliaire d'origine animale. La demanderesse a en effet montré que, de manière surprenante, un virus d'origine animale était capable de trans-complémenter un AAV humain et, de ce fait, pouvait être utilisé comme virus auxiliaire pour la production d'AAV recombinants humains. Ce résultat est particulièrement avantageux puisque le virus animal utilisé comme virus auxiliaire n'est pas capable de se propager dans les cellules humaines et qu'aucune pathologie humaine n'a été observée consécutivement à une infection par un virus animal auxiliaire. De ce fait, même si les préparations d'AAV recombinants obtenues peuvent être contaminées par du virus auxiliaire, celui-ci ne pourra occasioner aucun effet secondaire indésirable in vivo. De plus, le procédé de l'invention permet d'utiliser des lignées de cellules productrices animales qui sont dépourvues des effets tumorigènes connus pour les lignées humaines actuellement disponibles. L'invention repose également sur la mise au point de lignées cellulaires et de virus auxiliaires particulièrement avantageux pour la production d'AAV recombinants.

Un premier objet de l'invention réside donc dans un procédé de production d'AAV recombinants selon lequel la production est réalisée en présence d'un virus auxiliaire animal.
Plus préférentiellement, la production est réalisée dans une lignée cellulaire animale infectée par un virus auxiliaire animal.
L'invention concerne également, d'une manière générale, l'utilisation d'un virus auxiliaire animal pour la préparation d'AAV recombinants.

Comme indiqué ci-avant, le terme virus auxiliaire désigne au sens de la présente invention tout virus capable d'apporter en trans les fonctions nécessaires à la réplication des AAV. Le virus auxiliaire utilisable dans le procédé de l'invention peut être un virus d'origine canine, bovine, murine, ovine, porcine, aviaire ou encore simienne. Il est préférentiellement choisi parmi les adénovirus, les virus de l'herpes ou les virus de la vaccine.
De manière particulièrement avantageuse, on utilise dans le cadre de la présente invention un adénovirus d'origine animale. Les adénovirus d'origine animale utilisables dans le cadre de la présente invention peuvent être d'origine diverses, et en particulier, d'origine canine, bovine, murine, (exemple : Mav1, Beard et al., Virology 75 (1990) 81), ovine, porcine, aviaire ou encore simienne (exemple : SAV).
Plus particulièrement, parmi les adénovirus aviaires, on peut citer les sérotypes 1 à 10 accessibles à l'ATCC, comme par exemple les souches Phelps (ATCC VR-432), Fontes (ATCC VR-280), P7-A (ATCC VR-827), IBH-2A (ATCC VR-828), J2-A (ATCC VR-829), T8-A (ATCC VR-830), K-11 (ATCC VR-921) ou encore les souches référencées ATCC VR-831 à 835.
Parmi les adénovirus bovins, on peut utiliser les différents sérotypes connus, et notamment ceux disponibles à l'ATCC (types 1 à 8) sous les référencesATCC VR-313, 314, 639-642, 768 et 769.
On peut également utiliser les adénovirus murins FL (ATCC VR-550) et E20308 (ATCC VR-528), l'adénovirus ovin type 5 (ATCC VR-1343), ou type 6 (ATCC VR-1340); l'adénovirus porcin 5359), ou les adénovirus simiens tels que notamment les adénovirus référencée à l'ATCC sous les numéros VR-591-594, 941-943, 195-203, etc.
De préférence, on utilise dans le cadre de la présente invention, comme virus auxiliaire, des adénovirus d'origine canine, et, plus préférentiellement, toutes les souches des adénovirus CAV1 et CAV2 [souche manhattan ou A26/61 (ATCC VR-800) par exemple]. Les adénovirus canins ont fait l'objet de nombreuses études structurales. Ainsi, des cartes de restriction complètes des adénovirus CAV1 et CAV2 ont été décrites dans l'art antérieur (Spibey et al., J. Gen. Virol. 70 (1989) 165), et les gènes E1a, E3 ainsi que les séquences ITR ont été clonés et séquencés (voir notamment Spibey et al., Virus Res. 14 (1989) 241; Linné, Virus Res. 23 (1992) 119, WO 91/11525). Comme le montrent les exemples ci-après, les adénovirus canins sont capables de trans-complémenter les AAV pour leur réplication avec une efficacité particulièrement importante.

Ces différents virus d'origine animale peuvent être obtenus, par exemple, à partir de souches déposées dans des collections, puis amplifiés dans des lignées cellulaires compétentes, et éventuellement modifiés. Des techniques de production, isolation et modification d'adénovirus, ou de virus de l'herpès ont été décrites dans la littérature et peuvent être utilisées dans le cadre de la présente invention [Akli et al., Nature Genetics 3 (1993) 224; Stratford-Perricaudet et al., Human Gene Thecapy 1 (1990) 241: EP 185 573, Levrero et al., Gene 101 (1991) 195 ; Le Gal la Salle et al., Science 259 (1993) 988 ; Roemer et Friedmann, Eur. J. Biochem. 208 (1992) 211 ; Dobson et al., Neuron 5 (1990) 353 ; Chiocca et al., New Biol. 2 (1990) 739 ; Miyanohara et al., New Biol. 4 (1992) 238 ; WO91/18088, WO90/09441, WO88/10311, WO91/11525]. Ces différents virus peuvent ensuite être modifiés, par exemple par délétion, substitution, addition, etc. En particulier, ces virus peuvent dans certains cas être modifiés pour devenir défectifs pour leur réplication.

Comme indiqué ci-avant, la production des AAV recombinants selon le procédé de la présente invention est préférentiellement réalisée dans une lignée cellulaire animale infectée par le virus auxiliaire.
Généralement, la lignée cellulaire et le virus auxiliaire utilisés sont de la même espèce. A cet égard, différentes lignées cellulaires animales utilisables dans le cadre de la présente invention ont été décrites dans la littérature. Ainsi, parmi les lignées canines, on peut citer avantageusement la lignée de cellules rénales de lévrier GHK (Flow laboratories) ou la lignée cellulaire MDCK (ATCC n° CRL6253). Les conditions de culture de ces cellules et de préparation des virus ou de l'ADN viral ont été décrites dans la littérature (voir notamment Macatney et al., Science 44 (1988) 9; Fowlkes et al., J. Mol. Biol. 132 (1979) 163). D'autres lignées animales (par exemple accessibles dans des collections) peuvent également être utilisées.

Avantageusement, le procédé de l'invention met en oeuvre une lignée cellulaire canine infectée par un adénovirus canin.
Plus préférentiellement, le procédé de l'invention est réalisé avec, comme lignée cellulaire canine, la lignée MDCK ou GHK, ou un dérivé de celles-ci. On entend par lignée dérivée toute lignée obtenue à partir de ces lignées et conservant leur capacité à produire des AAV recombinants lorsqu'elles sont cultivées, infectées et/ou transfectées dans les conditions appropriées. Des lignées dérivées peuvent notamment être obtenues par insertion de gènes exogènes, mutagénèse, et/ou sélection de sous-clones, le cas échéant.

Dans un mode préféré de mise en oeuvre de l'invention, la lignée cellulaire utilisée possède en outre les fonctions d'encapsidation d'AAV.
Comme indiqué avant, les AAV recombinants sont généralement produits par cotransfection, dans une lignée cellulaire infectée par un virus auxiliaire, d'un plasmide contenant le gène d'intérêt bordé de deux régions répétées inversées (ITR) d'AAV et d'un plasmide portant les fonctions d'encapsidation d'AAV. Le fait de déléter les fonctions d'encapsidation de l'AAV recombinant et de les apporter en trans sur un plasmide permet en effet de préparer des AAV recombinants défectifs, c'est-à-dire incapables de produire, après infection dans une cellule cible, des particules virales infectieuses. Les fonctions d'encapsidation d'AAV sont constituées essentiellement par les gènes rep et cap, ou par tout gène homologue fonctionnel. Le gène rep est impliqué dans la réplication virale ainsi que dans l'expression des gènes viraux, et le gène cap code pour les protéines d'enveloppe du virus (VP1, VP2 et VP3). Ces gènes ont été caractérisés et leur séquence a été décrite dans la littérature (Srivastava et al., J. Virol. 45 (1983) 555). Un homologue fonctionnel correspond à tout gène obtenu par modification (mutation, suppression, addition, etc) des gènes rep ou cap et présentant une activité de même nature. De tels gènes homologues fonctionnels peuvent également être des gènes obtenus par hybridation à partir de banques d'acides nucléiques au moyen de sondes correspondant aux gènes rep ou cap.
Pour permettre la production d'AAV recombinants défectifs, la lignée cellulaire selon l'invention possède donc avantageusement les fonctions d'encapsidation d'AAV. Plus préférentiellement, la lignée cellulaire utilisée possède une ou plusieurs copies des gènes rep et cap d'AAV, ou de gènes homologues fonctionnels.

Selon la présente invention, les fonctions d'encapsidation d'AAV peuvent être apportées par un plasmide d'encapsidation et/ou par le virus auxiliaire animal utilisé et/ou être intégrées au génome de la lignée cellulaire.

Dans un mode de réalisation particulier de l'invention, toutes les fonctions d'encapsidation sont apportées par un plasmide dit d'encapsidation. Dans ce cas, le procédé de l'invention comprend avantageusement la co-transfection d'une lignée cellulaire animale infectée par un virus auxiliaire animal, par un vecteur portant un gène d'intérêt et au moins une ITR d'AAV et par ledit plasmide d'encapsidation d'AAV. Plus préférentiellement, le plasmide d'encapsidation d'AAV porte au moins les gènes rep et cap d'AAV ou des homologues fonctionnels.

Dans un autre mode de réalisation particulièrement avantageux de l'invention, les fonctions d'encapsidation sont apportées par le virus auxiliaire animal utilisé et/ou sont intégrées au génome de la lignée cellulaire. Dans ce cas, le procédé de l'invention comprend la transfection d'un vecteur portant un gène d'intérêt et au moins une ITR d'AAV dans une lignée cellulaire infectée par un virus auxiliaire animal et contenant les fonctions d'encapsidation (intégrées au génome et/ou portées par le virus auxiliaire). Ce mode de réalisation est particulièrement avantageux car il permet d'éviter l'emploi d'un plasmide d'encapsidation.

Plus préférentiellement, dans le procédé de l'invention, le gène rep d'AAV, ou un homologue fonctionnel de celui-ci, est intégré au génome de la lignée cellulaire et le gène cap d'AAV, ou un homologue fonctionnel de celui-ci, est porté par le virus auxiliaire animal. Dans une autre variante préférée de mise en oeuvre de l'invention, on utilise une lignée cellulaire comprenant, insérés dans son génome, les gènes rep et cap d'AAV ou des gènes homologues fonctionnels. Dans ce mode préféré, les gènes rep et cap d'AAV ou leurs homologues fonctionnels peuvent être introduits sur une construction unique ou, séquentiellement, sur des constructions séparées. Dans ces différents modes de réalisation, les fonctions d'encapsidation peuvent être placées sous le contrôle de leur propre promoteur, de promoteurs hétérologues ou de promoteurs artificiels. Avantageusement, les fonctions d'encapsidation sont placées sous le contrôle d'éléments de régulation de la transcription inductibles, permettant d'induire ou de réprimer l'expression des fonctions d'encapsidation selon les conditions. De manière particulièrement avantageuse, dans les constructions de l'invention, le gène rep d'AAV, ou un gène homologue fonctionnel, est placé sous le contrôle d'un promoteur inductible.
Toujours dans une autre variante préférée de mise en oeuvre de l'invention, on utilise un virus auxiliaire comprenant, dans son génome, les gènes rep et cap d'AAV ou des gènes homologues fonctionnels.
La demanderesse a en effet montré qu'il est possible d'insérer les fonctions d'encapsidation, ou certaines d'entre-elles, directement dans le génome du virus auxiliaire utilisé. De la même manière, la demanderesse a également montré qu'il est possible d'insérer les fonctions d'encapsidation, ou certaines d'entre-elles, de manière stable, directement dans le génome de la lignée cellulaire utilisée.

A cet égard, l'invention a également pour objet toute lignée cellulaire animale infectable par un virus auxiliaire pour la production d'AAV recombinants, comportant tout ou partie des fonctions d'encapsidation d'AAV intégrées dans son génome. Préférentiellement, l'invention concerne toute lignée cellulaire animale comportant le gène rep d'AAV ou un gène homologue fonctionnel, intégré dans son génome. Dans un autre mode préféré, l'invention concerne toute lignée cellulaire animale comportant les gènes rep et cap d'AAV ou des gènes homologues fonctionnels, intégrés dans son génome.
De manière particulièrement avantageuse, il s'agit d'une lignée cellulaire canine, telle que par exemple une lignée obtenue à partir des lignées MDCK ou GHK.

Les lignées cellulaires de l'invention peuvent être obtenues par transfection d'un fragment d'ADN portant la ou les fonctions d'encapsidation d'AAV sous contrôle de signaux d'expression. La transfection peut être réalisée par toute technique connue de l'homme du métier (précipitation au phosphate de calcium, lipofection. électroporation, utilisation de liposornes, etc). Préférentiellement, la transfection est réalisée en présence de phosphate de calcium (voir exemple 5). Pour contrôler l'expression des fonctions d'encapsidation, différents signaux d'expression peuvent être utilisés. Il peut s'agir en particulier des propres promoteurs des gènes rep ou cap, de promoteurs hétérologues ou même synthétiques. Dans un mode de réalisation particulièrement intéressant, dans les lignées cellulaires selon l'invention, les fonctions d'encapsidation d'AAV sont placées sous le contrôle de promoteurs inductibles (LTR-MMTV, Tc, etc). L'emploi de tels promoteurs est particulièrement avantageux puis qu'il permet de moduler l'effet du gène rep notamment sur le virus auxiliaire utilisé. L'invention a donc également pour objet toute lignée cellulaire infectable par un virus auxiliaire pour la production d'AAV recombinants, comportant tout ou partie des fonctions d'encapsidation d'AAV intégrées dans son génome, sous le contrôle d'un ou plusieurs promoteurs inductibles.
Dans le cas d'une lignée comportant les gènes rep et cap d'AAV (ou des homologues fonctionnels), ces gènes peuvent être transfectés soit sur une construction unique, soit, de manière séquentielle, sur deux constructions séparées. Ce deuxième mode de préparation est particulièrement avantageux puisqu'il limite au maximum les événements de recombinaison. Les lignées ainsi obtenues sont donc particulièrement stables. Par ailleurs, de manière avantageuse, la construction utilisée pour la transfection porte également un gène permettant la sélection des cellules transformées, et par exemple le gène de la résistance à la généticine. Le gène de résistance peut également être porté par un fragment d'ADN différent, co-transfecté avec le premier.
Après transfection, les cellules tranformées sont sélectionnées (par exemple pour leur résistance à la généticine) et leur ADN est analysé par Northen blot pour vérifier l'intégration du ou des fragments d'ADN (dans le cas de l'utilisation de 2 constructions séparées) dans le génome. La capacité des cellules obtenues à encapsider des AAV peut ensuite être testée par infection au moyen d'un AAV recombinant dépourvu desdites fonctions.

L'invention a également pour objet tout virus auxiliaire pour la production d'AAV recombinants comprenant, inséré dans son génome, tout ou partie des fonctions d'encapsidation des AAV. Plus préférentiellement, l'invention concerne tout virus auxiliaire pour la production d'AAV recombinants comprenant, inséré dans son génome, le gène rep d'AAV ou un gène homologue fonctionnel. Toujours préférentiellement, l'invention concerne tout virus auxiliaire pour la production d'AAV recombinants comprenant, insérés dans son génome, les gènes rep et cap d'AAV ou des gènes homologues fonctionnels.
Avantageusement, le virus auxiliaire pour la production d'AAV recombinants est un adénovirus. Les adénovirus comprenant les fonctions d'encapsidation des AAV selon l'invention peuvent être préparés par recombinaison homologue entre un adénovirus et un plasmide portant entre autre lesdites fonctions d'encapsidation. La recombinaison homologue se produit après co-transfection desdits adénovirus et plasmide dans une lignée cellulaire appropriée. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %), ou la lignée MDCK. Ensuite, les vecteurs qui se sont multipliés sont récupérés et purifiés selon les techniques classiques de biologie moléculaire.

Encore plus particulièrement, l'adénovirus est un adénovirus d'origine animale, de préférence choisi parmi les adénovirus canins (CAV1 et CAV2). Les fonctions d'encapsidation d'AAV peuvent être introduites dans différentes régions du génome du virus auxiliaire de l'invention. Avantageusement, les fonctions d'encapsidation sont insérées dans une région ne perturbant pas la capacité du virus de transcomplémenter les AAV. Il est également possible d'insérer les fonctions d'encapsidation dans une région fonctionnelle du génome du virus auxiliaire, laquelle région étant alors apportée en trans, soit par un plasmide, soit par la lignée cellulaire utilisée. Ainsi, il est possible par exemple d'insérer le gène rep, le gène cap ou les gènes rep et cap en remplacement du gène E1. L'adénovirus obtenu peut être utilisé comme virus auxiliaire pour la production d'AAV recombinants dans la lignée cellulaire 293, qui porte le gène E1 dans son génome (voir exemple 6).

Le procédé de l'invention permet ainsi la production d'AAV recombinants utilisables thérapeutiquement. Les AAV recombinants obtenus peuvent être tout AAV dont le génome au moins a été modifié par insertion d'un gène d'intérêt. Comme indiqué ci-avant, différents types d'AAV recombinants ont été décrits dans l'art antérieur (WO 91/18088; WO 93/09239; US 4,797,368, US5,139,941, EP 488 528). Généralement, ces AAV comprennent des délétions partielles ou totales dans les gènes rep et/ou cap, au niveau desquelles un gène d'intérêt est inséré. Ces AAV recombinants peuvent être préparés à partir des différents sérotypes d'AAV, tels que les AAV1, AAV2, AAV3 et AAV4, et, préférentiellement, à partir des souches AAV2. Concernant le gène d'intérêt, il peut s'agir en particulier d'un gène thérapeutique.
Au sens de l'invention, on entend par gène thérapeutique notamment tout gène codant pour un produit protéique ayant un effet thérapeutique. Le produit protéique ainsi codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.
Parmi les produits thérapeutiques au sens de la présente invention, on peut citer plus particulièrement les enzymes, les dérivés sanguins, les hormones, les lymphokines : interleukines, interférons, TNF, etc (FR 9203120), les facteurs de croissance (érythropoiétine, G-CSF, M-CSF, GM-CSF, etc), les neurotransmetteurs
ou leurs précurseurs ou enzymes de synthèse, les facteurs trophiques : BDNF, CNTF, NGF, IGF, GMF, aFGF, bFGF. NT3, NT5, HARP/pléiotrophine, etc; les apolipoprotéines : ApoAI. ApoAIV, ApoE, etc (FR 93 05125), la lipoprotéine lipase (LPL), la dystrophine ou une minidystrophine (FR 9111947), la protéine CFTR associée à la mucoviscidose, les gènes suppresseurs de tumeurs : p53: Rb, Rap1A. DCC, k-rev, etc (FR 93 04745), les gènes codant pour des facteurs impliqués dans la coagulation : Facteurs VII, VIII, IX, les gènes intervenant dans la réparation de l'ADN, les gènes suicides (thymidine kinase, cytosine déaminase), etc.

Le gène thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les antisens comprennent également les séquences codant pour des ribozymes, qui sont capables de détruire sélectivement des ARN cibles (EP 321 201).

Le gène d'intérêt peut également comporter une ou plusieurs séquences codant pour un peptide antigénique, capable de générer chez l'homme ou l'animal une réponse immunitaire. Dans ce mode particulier de mise en oeuvre de l'invention, les AAV recombinants peuvent être utilisés pour la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, ou encore spécifiques de tumeurs (EP 259 212).

Préférentiellement, le gène d'intérêt comprend également des séquences permettant son expression dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, LTR-RSV, etc. En outre, ces séquences d'expression peuvent être modifiées par addition de séquences d'activation, de régulation, ou conférant au gène d'intérêt une spécificité d'expression tissulaire.
Par ailleurs, le gène d'intérêt peut également comporter une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle.

Les AAV recombinants préparés selon la présente invention peuvent être utilisés pour le transfert de gènes d'intérêt in vitro, ex vivo ou in vivo. In vitro, ils peuvent permettre de transférer un gène à une lignée cellulaire, par exemple en vue de produire une protéine recombinante. Ex vivo, ils peuvent être utilisés pour transférer un gène sur une population de cellules prélevée à partir d'un organisme, éventuellement sélectionnée et amplifiée, dans le but de conférer à ces cellules des propriétés désirées en vue de leur réadministration à un organisme. In vivo, ils peuvent être utilisés pour le transfert de gènes par administration directe d'une solution purifiée, éventuellement associée à un ou plusieurs véhicules pharmaceutiques. Dans ce dernier cas, les AAV recombinants peuvent être formulés en vue d'administrations par voie topique, cutanée, orale, rectale, vaginale, parentérale, intranasale, intraveineuse, intramusculaire, sous-cutanée, intraoculaire, transdennique, etc. De préférence, ils sont associés à un véhicule pharmaceutiquement acceptable pour une formulation injectable, notamment pour une injection directe au niveau de l'organe désiré. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'AAV utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée.

La présente invention fournit ainsi une méthode particulièrement avantageuse pour la préparation d'AAV recombinants utilisables thérapeutiquement, notamment dans une méthode de traitement de maladies, comprenant l'administration in vivo d'un AAV recombinant contenant un gène apte à corriger ladite maladie. Plus particulièrement, cette méthode est applicable aux maladies résultant d'une déficience en un produit protéique ou nucléique et le gène d'intérêt code pour ledit produit protéique ou contient ledit produit nucléique.

La présente invention sera plus complètement décrite à l'aide des exemples qui suivent, qui doivent être considérés comme illustratifs et non limitatifs.

### Techniques générales de Biologie Moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds). "Current Protocols in Molecular Biology", John Wiley & Sons, New York, 1987].
Les plasmides de type pBR322, pUC et les phages de la série M13 sont d'origine commerciale (Bethesda Research Laboratories).
Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.
Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.
La mutagénèse dirigée in vitro par oligodéoacynucléotides synthétiques peut être effectuée selon la méthode développée par Taylor et al. [Nucleic Acids Res. 13 (1985) 8749-8764] en utilisant le kit distribué par Amersham.
L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.
La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Légende des figures

Figure 1 : Représentation du plasmide pREP-CAP
Figure 2 : Représentation du plasmide pAAV-EPO-NEO
Figure 3 : Représentation du plasmide pAd-RepCap

### EXEMPLE 1 - Construction du plasmide d'encapsidation pREP-CAP

Cet exemple décrit la construction d'un plasmide dit d'encapsidation portant les fonctions nécessaires à l'encapsidation d'un AAV recombinant. Plus précisément, ce plasmide, désigné pREP-CAP porte les gènes rep et cap de l'AAV2.

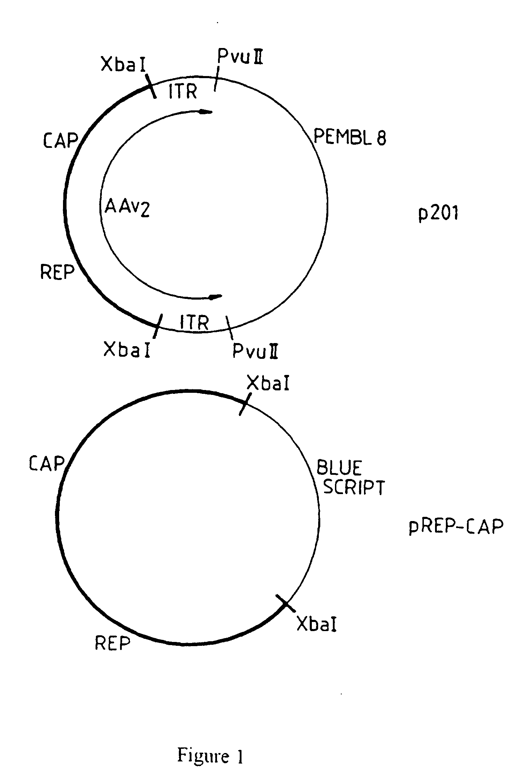

Le plasmide pREP-CAP a été construit de la manière suivante : Le génome de l'AAV2 délété de ses ITR a été isolé a partir du plasmide p201, par digestion au moyen de l'enzyme XbaI. Le plasmide p201 contient l'ensemble du génome de l'AAV2 cloné au site PvuII du plasmide pEMBL8 (Samulski et al., J. Virol. 61 (1987) 3096). Le fragment ainsi obtenu, portant les gènes rep et cap a ensuite été cloné au site XbaI du plasmide Bluescript (Stratagene). La structure de ce plasmide est représentée sur la figure 1.

### EXEMPLE 2 - Construction du plasmide AAV pAAV-EPO-NEO

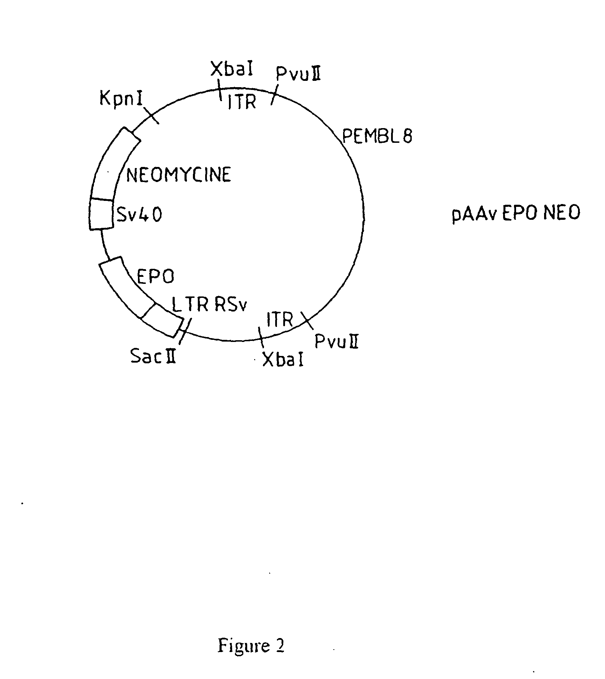

Cet exemple décrit la construction d'un plasmide AAV portant le cDNA codant pour l'érythropoiétine, un gène marqueur, et deux ITR d'AAV2. Ce plasmide a été désigné pAAV-EPO-NEO (figure 2).

Pour la construction de ce plasmide, une cassette d'expression a été préparée contenant le cDNA codant pour l'érythropoiétine (cynomolgus) sous contrôle du LTR du virus du sarcome de rous (RSV), suivi du gène neo conférant la résistance à la néomycine sous controle du promoteur précoce du virus SV40. Cette cassette a ensuite été insérée aux sites SacII-KpnI du plasmide p201, en remplacement du fragment correspondant contenant les gènes rep et cap (figure 2).

### EXEMPLE 3 - Production d'adénovirus canin CAV2 dans la lignée cellulaire MDCK

Cet exemple décrit la production d'adénovirus canin CAV2 dans la lignée cellulaire canine MDCK.

Les cellules MDCK ont été cultivées dans un milieu DMEM supplémenté par 10 % de sérum de veau (voir aussi Mcatney et al., Science 44 (1988) 9 pour les conditions de culture). Les cellules à confluence ont ensuite été infectées par une suspension de virus CAV2 (environ 5 pfu/cellule). 30 à 48 heures après l'infection, les cellules infectées ont été récoltées et concentrées par centrifugation. Le culot obtenu peut être utilisé pour purifier les adénovirus CAV2 sur chlorure de césium. Toutefois, si les titres sont trop faibles, le culot est préférentiellement soumis à un cycle de congélation-décongélation, puis le surnageant obtenu est utilisé pour amplifier les virus CAV2. Pour cela, le surnageant est utilisé pour réinfecter, dans les mêmes conditions, les cellules MDCK, puis le protocole décrit ci-dessus pour la récupération des virus est répété. Ce procédé permet d'obtenir une solution purifiée d'adénovirus CAV2 utilisable comme virus auxiliaire pour la production d'AAV recombinants.

### EXEMPLE 4 - Production d'AAV recombinant portant le gène de l'érythropoiétine dans la lignée cellulaire MDCK infectée par un adénovirus auxiliaire canin.

Cet exemple décrit la production d'un AAV recombinant portant le gène de l'érythropoiétine dans la lignée cellulaire MDCK infectée par l'adénovirus canin CAV2.

Les cellules MDCK (50 % de la confluence) ont été infectées avec 5-10 pfu/cellule d'adénovirus CAV2 préparé dans les conditions de l'exemple 3. Quatre à 6 heures après l'infection, les cellules ont été co-transfectées par lipofection (Fegner et al., PNAS 84 (1987) 7413) avec 5 µg de plasmide pAAV-EPO-NEO et 5 µg de plasmide pREP-CAP. 48 à 72 heures après, les cellules ont été récoltées et soumises à plusieurs cycles de congélation-décongélation. La suspension obtenue a ensuite été centrifugée (15 minutes à 4000 tour/min), puis le surnageant contenant les AAV recombinants a été récupéré et chauffé à 56°C pendant 30 minutes.
Pour vérifier le pouvoir infectieux et l'expression de l'érythropoiétine, des échantillons du surnageant ont été utilisés pour infecter des cellules Hela. Les cellules Hela sont issues d'un carcinome de l'épithelium humain. Cette lignée est accessible à l'ATCC (ref. CCL2) ainsi que les conditions permettant sa culture. 24 heures après l'infection, les cellules et le surnageant ont été récoltés. La présence du génome de l'AAV-EPO recombinant dans ces cellules a ensuite été confirmée par des expériences d'hybridation en "southern blot" sur l'ADN cellulaire extrait selon la technique de Hirt (Gluzman et Van Doren, J. Virol. 45 (1983) 91). De plus, la présence d'érythropoiétine dans le surnageant a été démontrée par test biologique.
Ces résultats montrent clairement que des AAV recombinants peuvent être préparés efficacement dans un système animal (lignée cellulaire et virus auxiliaire).

### EXEMPLE 5 - Construction de lignées cellulaires contenant les fonctions d'encapsidation d'AAV.

Cet exemple décrit la construction de lignées cellulaires contenant tout ou partie des fonctions d'encapsidation d'AAV. Ces lignées permettent la construction d'AAV recombinants selon l'invention délétés pour ces régions, sans avoir recours à un plasmide d'encapsidation. Ces virus sont obtenus par recombinaison in vivo, et peuvent comporter des séquences hétérologues importantes.
Les lignées de l'invention sont construites par transfection des cellules correspondantes par un fragment d'ADN portant les gènes indiqués sous le controle d'un promoteur de la transcription et d'un terminateur (site de polyadénylation).
La transfection peut être réalisée de différentes manières, et notamment en présence de phosphate de calcium, par lipofection, electroporation, etc. Dans cet exemple, la transfection est réalisée en présence de phosphate de calcium.
Le terminateur peut être soit le terminateur naturel du gène transfecté, soit un terminateur différent comme par exemple le terminateur du messager précoce du virus SV40.
Dans les lignées cellulaires décrites, les gènes rep et/ou cap sont placés sous le controle d'un promoteur inductible : le LTR de MMTV (Pharmacia), qui est induit par la dexaméthasone. Il est entendu que d'autres promoteurs peuvent être utilisés, et notamment des variants du LTR de MMTV portant par exemple des régions hétérologues de régulation (région "enhancer" notamment), ou le promoteur tétracycline (Tc). Par ailleurs, comme indiqué dans la description, le promoteur peut également être le propre promoteur du gène utilisé.
Avantageusement, le fragment d'ADN porte également un gène permettant la sélection des cellules transformées. Il peut s'agir en particulier d'un gène de résistance à un antibiotique, tel que par exemple le gène de la résistance à la généticine. Ce gène marqueur peut également être porté par un fragment d'ADN différent, co-transfecté avec le premier.
Après transfection, les cellules tranformées sont sélectionnées (résistance à la généticine) et leur ADN est analysé par Northen blot pour vérifier l'intégration du ou des fragments d'ADN (dans le cas de l'utilisation de 2 constructions séparées) dans le génome. Les cellules sont ensuite testées pour leur capacité à encapsider des AAV recombinants après infection par le virus auxiliaire et transfection avec un plasmide AAV appropriés.
Cette technique permet d'obtenir les lignées cellulaires suivantes :
1. Cellules MDCK possédant le gène rep sous controle du LTR de MMTV;
2. Cellules MDCK possédant les gènes rep et cap , sur un même fragment d'ADN, sous controle du LTR de MMTV;
3. Cellules MDCK possédant les gènes rep et cap , sur 2 fragments d'ADN distincts, sous controle du LTR de MMTV

### EXEMPLE 6 - Construction d'un adénovirus contenant les fonctions d'encapsidation d'AAV.

Cet exemple décrit la construction d'un adénovirus contenant les gènes rep et cap d'AAV insérés dans son génome. L'adénovirus Ad-RSV.Rep-Cap a été obtenu par recombinaison homologue in vivo entre l'adénovirus mutant Ad.dl1324 [Thimmappaya et al., Cell 31 (1982) 543] et le vecteur pAd.RSV.Rep-Cap.

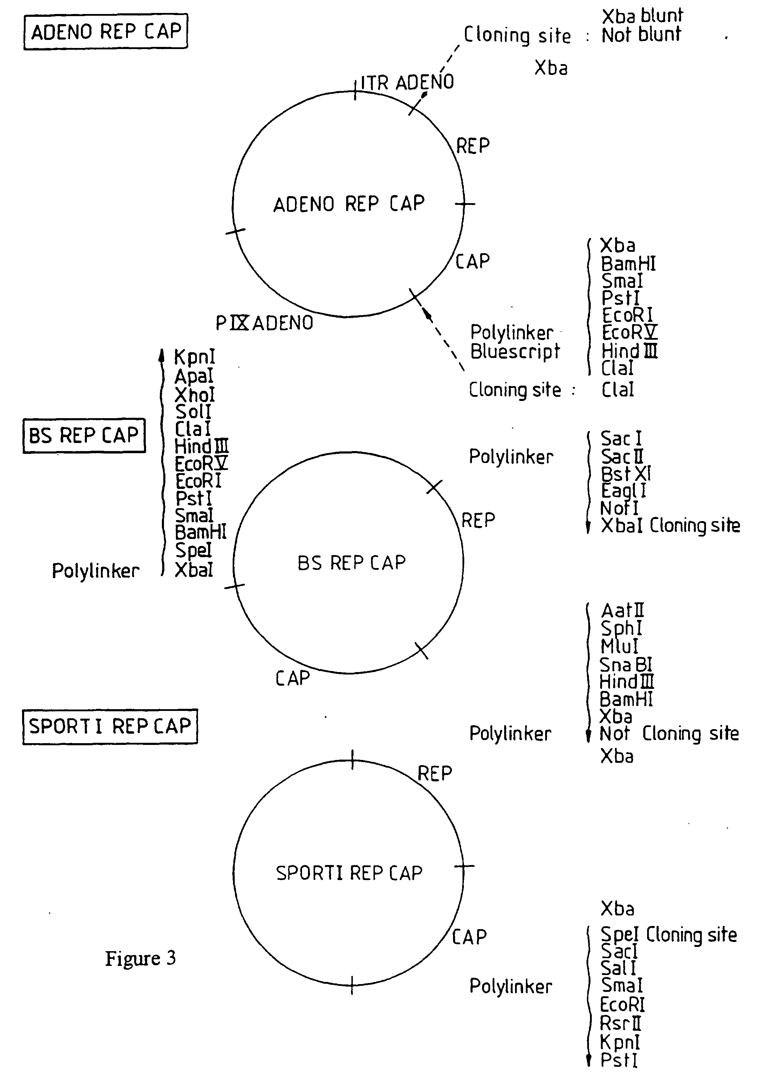

### 6.1. Construction du plasmide pAd.RSV.Rep-Cap (figure 3)

Le plasmide pAd.RSV.Rep-Cap a été construit à partir du plasmide pAd.RSV.βGal [Stratford-Perricaudet et al., J. Clin. Invest. (1992) 626] par substitution du gène β-gal par un fragment d'ADN portant les gènes rep et cap d'AAV.
Pour cela, le fragment XbaI contenant le génome de l'AAV2 délété de ses ITR a été isolé à partir du plasmide p201 (Cf exemple 1). Ce fragment a ensuite été sous-cloné au site XbaI du plasmide Bluescript. Cette étape permet ensuite d'isoler le fragment comportant les gènes rep et cap d'AAV sous forme d'un fragment NotI-ClaI. Ce fragment a ensuite été cloné aux sites XbaI (situé après l'ITR gauche) et ClaI (situé avant PIX de l'Ad5) du vecteur pAd.RSV.βGal, résultant en la substitution du gène βgal par les gènes rep et cap.

### 6.2. Construction de l'adénovirus recombinant défectif Ad.RSV.Rep-Cap

Le plasmide pAd.RSV.Rep-Cap et l'adénovirus Ad.d11324 linéarisé par l'enzyme ClaI, sont co-transfectés dans la lignée 293 en présence de phosphate de calcium, pour permettre la recombinaison homologue. Les adénovirus recombinants ainsi générés sont sélectionnés par purification sur plaque. Après isolement, l'ADN de l'adénovirus recombinant est amplifié dans la lignée cellulaire 293.
Les particules virales sont généralement purifiées par centrifugation sur gradient de chlorure de césium selon les techniques connues (voir notamment Graham et al., Virology 52 (1973) 456). L'adénovirus Ad.RSV.Rep-Cap peut être conservé à -80°C dans 20 % de glycérol.
La même stratégie peut être répétée en insérant seulement l'un des gènes rep ou cap. Par ailleurs, l'insertion peut être réalisée en des sites différents du génome de l'adénovirus.

## Revendications

1. Procédé de production de virus adéno-associés (AAV) humains recombinants **caractérisé en ce que** la production est réalisée dans une lignée cellulaire en présence d'un virus auxiliaire animal non humain.

2. Procédé selon la revendication 1, **caractérisée en ce que** la production est réalisée dans une lignée cellulaire animale infectée par un virus auxiliaire animal non humain.

3. Procédé selon la revendication 1 ou 2 **caractérisé en ce que** le virus auxiliaire animal non humain est un virus d'origine canine, bovine, murine, ovine, porcine, aviaire ou simienne.

4. Procédé selon la revendication 3 **caractérisé en ce que** le virus auxiliaire animal non humain est choisi parmi les adénovirus, les virus de l'herpès et les virus de la vaccine.

5. Procédé selon la revendication 4 **caractérisé en ce que** le virus auxiliaire animal non humain est un adénovirus.

6. Procédé selon la revendication 5 **caractérisé en ce que** le virus auxiliaire animal non humain est un adénovirus canin.

7. Procédé selon la revendication 6 **caractérisé en ce que** le virus auxiliaire animal non humain est choisi parmi les souches CAV-1 et CAV-2.

8. Procédé selon les revendications 6 ou 7 **caractérisé en ce que** la production est réalisée dans une lignée cellulaire canine.

9. Procédé selon la revendication 8 **caractérisé en ce que** la lignée cellulaire est la lignée MDCK ou GHK ou un dérivé de celles-ci.

10. Procédé selon l'une quelconque des revendications précédentes **caractérisé en ce que** la lignée cellulaire possède en outre les fonctions d'encapsidation d'AAV.

11. Procédé selon la revendication 10 **caractérisé en ce que** les fonctions d'encapsidation d'AAV sont constituées par les gènes rep et cap d'AAV ou des homologues fonctionnels, éventuellement placés sous le contrôle d'éléments hétérologues de régulation de la transcription.

12. Procédé selon la revendication 10 ou 11 **caractérisé en ce que** les fonctions d'encapsidation d'AAV sont apportées par un plasmide d'encapsidation et/ou par le virus auxiliaire animal non humain et/ou sont intégrées au génome de la lignée cellulaire.

13. Procédé selon la revendication 12 **caractérisé en ce que** le gène rep d'AAV, ou un homologue fonctionnel de celui-ci, est intégré au génome de la lignée et le gène cap d'AAV, ou un homologue fonctionnel de celui-ci, est porté par le virus auxiliaire animal non humain.

14. Procédé selon la revendication 12 **caractérisé en ce que** les gènes rep et cap d'AAV ou des gènes homologues fonctionnels sont intégrés dans le génome de la lignée cellulaire

15. Procédé selon la revendication 12 **caractérisé en ce que** les gènes rep et cap d'AAV ou des gènes homologues fonctionnels sont portés par le virus auxiliaire.

16. Lignée cellulaire animale infectable par un virus auxiliaire animal non humain pour la production d'AAV humains recombinants, comportant les fonctions d'encapsidation d'AAV intégrées dans son génome.

17. Lignée cellulaire infectable par un virus auxiliaire animal non humain pour la production d'AAV recombinants humains, comportant les fonctions d'encapsidation d'AAV intégrées dans son génome, sous le contrôle d'un ou plusieurs promoteurs inductibles.

18. Lignée cellulaire selon la revendication 16 ou 17 **caractérisée en ce qu'**elle comporte le gène rep d'AAV ou un gène homologue fonctionnel intégré dans son génome.

19. Lignée cellulaire selon la revendication 16 ou 17 **caractérisée en ce qu'**elle comporte les gènes rep et cap d'AAV ou des gènes homologues fonctionnels intégrés dans son génome.

20. Lignée cellulaire selon la revendication 17 **caractérisée en ce que** le promoteur inductible est choisi parmi le LTR de MMTV ou le promoteur Tc.

21. Lignée cellulaire selon la revendication 16 **caractérisée en ce qu'**elle est obtenue à partir des lignées MDCK ou GHK.

22. Virus auxiliaire animal non humain pour la production d'AAV humains recombinants comprenant, inséré dans son génome, les fonctions d'encapsidation d'AAV.

23. Virus auxiliaire selon la revendication 22 **caractérisé en ce qu'**il comprend, inséré dans son génome, le gène rep d'AAV ou un gène homologue fonctionnel.

24. Virus auxiliaire selon la revendication 22 **caractérisé en ce qu'**il comprend, insérés dans son génome, les gènes rep et cap d'AAV ou des gènes homologues fonctionnels.

25. Virus auxiliaire selon l'une quelconque des revendications 22 à 24, **caractérisé en ce qu'**il s'agit d'un adénovirus animal non humain.

26. Virus auxiliaire selon la revendication 25 **caractérisé en ce qu'**il s'agit d'un adénovirus canin, de préférence choisi parmi les adénovirus CAV1 et CAV2.

## Patentansprüche

1. Herstellungsverfahren von menschlichen rekombinanten adeno-assoziierten Viren (AAV, **dadurch gekennzeichnet, dass** die Herstellung in einer Zelllinie bei Vorhandensein eines nicht menschlichen, tierischen Hilfsvirus realisiert wird.

2. Verfahren gemäß Anspruch 1, **dadurch gekennzeichnet, dass** die Herstellung in einer durch ein nicht menschliches, tierisches Hilfsvirus infizierten tierischen Zelllinie realisiert wird.

3. Verfahren gemäß Anspruch 1 oder 2, **dadurch gekennzeichnet, dass** das nicht menschliche, tierische Hilfsvirus ein aus einem Hund, einem Rind, einer Maus, einem Schaf, einem Schwein, Geflügel oder einem Affen stammendes Virus ist.

4. Verfahren gemäß Anspruch 3, **dadurch gekennzeichnet, dass** das nicht menschliche, tierische Hilfsvirus aus den Adenoviren, den Herpesviren und den Viren der Vakzine ausgewählt wird.

5. Verfahren gemäß Anspruch 4, **dadurch gekennzeichnet, dass** das nicht menschliche, tierische Hilfsvirus ein Adenovirus ist.

6. Verfahren gemäß Anspruch 5, **dadurch gekennzeichnet, dass** das nicht menschliche, tierische Hilfsvirus ein Hunde-Adenovirus ist.

7. Verfahren gemäß Anspruch 6, **dadurch gekennzeichnet, dass** das nicht menschliche, tierische Hilfsvirus aus den Stämmen CAV-1 und CAV-2 ausgewählt wird.

8. Verfahren gemäß Anspruch 6 oder 7, **dadurch gekennzeichnet, dass** die Herstellung in einer Hunde-Zelllinie realisiert wird.

9. Verfahren gemäß Anspruch 8, **dadurch gekennzeichnet, dass** die Zelllinie die Linie MDCK oder GHK oder ein Derivat davon ist.

10. Verfahren gemäß einem der vorherigen Ansprüche, **dadurch gekennzeichnet, dass** die Zelllinie darüber hinaus die Enkapsidationsfunktionen von AAV besitzt.

11. Verfahren gemäß Anspruch 10, **dadurch gekennzeichnet, dass** die Enkapsidationsfunktionen von AAV durch die Gene rep und cap von AAV oder eventuell der Kontrolle von heterologen Regulationselementen der Transkription unterstehende funktionale Homologe gebildet werden.

12. Verfahren gemäß Anspruch 10 oder 11, **dadurch gekennzeichnet, dass** die Enkapsidationsfunktionen von AAV durch ein Enkapsidationsplasmid und / oder durch das nicht menschliche, tierische Hilfsvirus erbracht werden und / oder in das Genom der Zelllinie integriert werden.

13. Verfahren gemäß Anspruch 12, **dadurch gekennzeichnet, dass** das Gen rep von AAV oder ein funktionales Homolog davon in das Genom der Linie integriert wird und das Gen cap von AAV oder ein funktionales Homolog davon, durch das nicht menschliche, tierische Virus getragen wird.

14. Verfahren gemäß Anspruch 12, **dadurch gekennzeichnet, dass** die Gene rep und cap von AAV oder funktionale homologe Gene in das Genom der Zelllinie integriert werden.

15. Verfahren gemäß Anspruch 12, **dadurch gekennzeichnet, dass** die Gene rep und cap von AAV oder funktionale homologe Gene durch das Hilfsvirus getragen werden.

16. Durch ein nicht menschliches, tierisches Hilfsvirus infizierbare, tierische Zelllinie für die Herstellung von rekombinanten menschlichen AAV mit in seinem Genom integrierten Enkapsidationsfunktionen von AAV.

17. Durch ein nicht menschliches, tierisches Hilfsvirus infizierbare Zelllinie für die Herstellung von menschlichen rekombinanten AAV mit unter der Kontrolle eines oder mehrerer induzierbarer Promoter in seinem Genom integrierten Enkapsidationsfunktionen von AAV.

18. Zelllinie gemäß Anspruch 16 oder 17, **dadurch gekennzeichnet, dass** sie das Gen rep von AAV oder ein in sein Genom integriertes funktionale homologes Gen umfasst.

19. Zelllinie gemäß Anspruch 16 oder 17, **dadurch gekennzeichnet, dass** sie die Gene rep und cap von AAV oder in sein Genom integrierte funktionale homologe Gene umfasst.

20. Zelllinie gemäß Anspruch 17, **dadurch gekennzeichnet, dass** der induzierbare Promoter aus dem LTR von MMTV oder dem Promoter Tc gewählt wird.

21. Zelllinie gemäß Anspruch 16, **dadurch gekennzeichnet**, das sie ausgehend von den Linien MDCK oder GHK gewonnen wird.

22. Nicht menschlicher, tierischer Hilfsvirus für die Herstellung von rekombinanten menschlichen AAVs mit den in seinem Genom eingeführten Enkapsidationsfunktionen von AAV.

23. Hilfsvirus gemäß Anspruch 22, **dadurch gekennzeichnet, dass** bei ihm das Gen rep von AAV oder ein funktionales homologes Gen in ein Genom eingefügt ist.

24. Hilfsvirus gemäß Anspruch 22, **dadurch gekennzeichnet, dass** bei ihm die Gene rep und cap von AAV oder funktionale homologe Gene in sein Genom eingefügt sind.

25. Hilfsvirus gemäß Anspruch 22 bis 24, **dadurch gekennzeichnet, dass** es sich um einen nicht menschlichen, tierischen Adenovirus handelt.

26. Hilfsvirus gemäß Anspruch 25, **dadurch gekennzeichnet, dass** es sich um einen bevorzugt aus den Adenoviren CAV1 und CAV2 ausgewählten Hunde-Adenovirus handelt.

## Claims

1. Method for the production of human recombinant adeno-associated viruses (AAV) **characterised in that** the production is carried out with a cell line in the presence of a non human animal auxiliary virus.

2. Method according to claim 1, **characterised in that** the production is carried out in an animal cell line infected by a non human animal auxiliary virus.

3. Method according to claim 1 or 2 **characterised in that** the non human animal auxiliary virus is a virus of canine, murine, ovine, porcine, bird or simian origin.

4. Method according to claim 3 **characterised in that** the non human animal auxiliary virus is chosen from among the adenoviruses, herpes viruses and vaccine viruses.

5. Method according to claim 4 **characterised in that** the non human animal auxiliary virus is an adenovirus.

6. Method according to claim 5 **characterised in that** the non human animal auxiliary virus is a canine adenovirus.

7. Method according to claim 6 **characterised in that** the non human animal auxiliary virus is chosen from among the strains CAV-1 and CAV-2.

8. Method according to claims 6 or 7 **characterised in that** the production is carried out in a canine cell line.

9. Method according to claim 8 **characterised in that** the cell line is line MDCK or GHK or a derivative of these lines.

10. Method according to any of the previous claims **characterised in that** the cell line also has AAV encapsulation functions.

11. Method according to claim 10 **characterised in that** the AAV encapsulation functions are formed by the AAV genes rep and cap or functional homologues, eventually placed under the control of heterologue elements of transcription regulation.

12. Method according to claim 10 or 11
**characterised in that** the AAV encapsulation functions are provided by an encapsidation plasmid and/or by the non human animal auxiliary virus and/or are integrated in the genome of the cell line.

13. Method according to claim 12 **characterised in that** AAV gene rep, or a functional homologue of it, is integrated in the genome of the line and AAV gene cap, or a functional homologue of it, is carried by the non human animal auxiliary virus.

14. Method according to claim 12 **characterised in that** AAV genes rep and cap or functional homologue genes are integrated in the genome of the cell line.

15. Metnod according to claim 12 **characterised in that** AAV genes rep and cap or functional homologue genes are carried by the auxiliary virus.

16. Animal cell line that can be infected by a non human animal auxiliary virus for the production of recombinant human AAV, comprising AAV encapsulation functions integrated in its genome.

17. Cell line that can be infected by a non human animal auxiliary virus for the production of recombinant human AAV, comprising AAV encapsulation functions integrated in its genome, under the control of one or several inductible promotors.

18. Cell line according to claim 16 or 17 **characterised in that** it comprises AAV gene rep or a homologous functional gene integrated in its genome.

19. Cell line according to claim 16 or 17 **characterised in that** it comprises AAV genes rep and cap or homologous functional genes integrated in its genome.

20. Cell line according to claim 17 **characterised in that** the inductible promotor is chosen from among MMTV LTR or promotor Tc.

21. Cell line according to claim 16 **characterised in that** it is obtained from lines MDCK or GHK.

22. Non human animal auxiliary virus for the production of recombinant human AAV comprising AAV encapsulation functions inserted in its genome.

23. Auxiliary virus according to claim 22 **characterised in that** it includes AAV gene rep or a functional homologue gene inserted in its genome.

24. Auxiliary virus according to claim 22 **characterised in that** it includes AAV genes rep and cap or functional homologue genes inserted in its genome

25. Auxiliary virus according to any of claims 22 to 24, **characterised in that** it consists of a non human animal adenovirus.

26. Auxiliary virus according to claim 25 **characterised in that** it consists of a canine adenovirus, preferably chosen from among adenoviruses CAV1 and CAV2.
